(19) Europäisches Patentamt
European Patent Office
Office européen des brevets

(11) **EP 2 332 499 B1**

(12) **EUROPÄISCHE PATENTSCHRIFT**

(45) Veröffentlichungstag und Bekanntmachung des Hinweises auf die Patenterteilung:
**27.01.2016 Patentblatt 2016/04**

(51) Int Cl.:
*A61F 2/90* *(2013.01)*    *A61F 2/86* *(2013.01)*

(21) Anmeldenummer: **10191148.5**

(22) Anmeldetag: **15.11.2010**

(54) **Endoprothese**

Endoprosthesis

Endoprothèse

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.12.2009 US 285549 P**

(43) Veröffentlichungstag der Anmeldung:
**15.06.2011 Patentblatt 2011/24**

(73) Patentinhaber: **Biotronik VI Patent AG**
**6341 Baar (CH)**

(72) Erfinder: **Schmiedl, Robert**
**96114, Hirschaid (DE)**

(74) Vertreter: **Galander, Marcus et al**
**Biotronik SE & Co. KG**
**Corporate Intellectual Properties**
**Woermannkehre 1**
**12359 Berlin (DE)**

(56) Entgegenhaltungen:
**EP-A1- 0 827 725      EP-A1- 1 642 551**
**WO-A1-2007/005800      US-A1- 2005 070 995**
**US-A1- 2008 051 866**

**Beschreibung**

Die Erfindung betrifft eine Endoprothese, insbesondere eine intraluminale Endoprothese, mit einer vorzugsweise hohlzylinderförmig ausgebildeten Grundstruktur, die vorzugsweise als Grundgitter ausgestaltet ist.

Sehr häufig verwendete Vertreter von Endoprothesen sind Stents (endovaskuläre Prothese), die zur Behandlung von Stenosen (Gefäßverengungen) eingesetzt werden. Stents weisen in der Regel eine hohlzylinder- oder rohrförmige Grundstruktur auf, welche in Längsrichtung an beiden Enden offen ist. Die Endoprothese wird in das zu behandelnde Gefäß oder Körperteil eingesetzt und dient dazu, dieses zu stützen und/oder offen zu halten. Stents nehmen üblicherweise zwei Zustände an, nämlich einen komprimierten Zustand mit einem kleinen Durchmesser und einen expandierten Zustand mit einem größeren Durchmesser. Im komprimierten Zustand kann der Stent mittels eines Katheters in das zu stützende Gefäß eingeführt und an der zu behandelnden Stelle positioniert werden. Hierfür ist der Stent häufig auf einen Katheter aufgecrimpt. Am Ort der Behandlung wird der Stent dann beispielsweise mittels eines Ballonkatheters dilatiert oder geht (bei Verwendung eines Formgedächtnismetalls als Stentmaterial) durch Erwärmung im Blut über seine Sprungtemperatur in den expandierten Zustand über.

Ursächlich für den Nutzen der Implantation von Endoprothesen in Gefäße ist der höhere primäre Lumengewinn, der durch das Innenvolumen der Grundstruktur erzeugt wird. Durch den Einsatz von derartigen Endoprothesen kann zwar ein für den Therapieerfolg notwendiger optimaler Gefäßquerschnitt erreicht werden, allerdings induziert die dauerhafte Anwesenheit eines solchen Fremdkörpers eine Kaskade von mikrobiologischen Prozessen, die zu einem allmählichen Zuwachsen des Stents führen kann. Beispielsweise werden beim Kontakt der Endoprothese mit der Gefäßwand während der Dilatation bzw. beim Aufweiten des Gefäßes kleinste Verletzungen, Einrisse oder Dissektionen der Gefäßwand verursacht, welche zwar zumeist problemlos verheilen, jedoch durch das ausgelöste Zellwachstum zu Wucherungen führen können. Auch die dauerhafte Anwesenheit eines Implantats bewirkt Prozesse, die zu einer Querschnittsverengung des Gefäßes bzw. Restenose führen können.

Aus oben angegebenen Gründen war es bisher Ziel der Entwicklung von Stents, eine möglichst hohe Radialfestigkeit mit möglichst geringem Recoil (elastische Rückfederung) zu erzielen. Hierbei wird unter Radialfestigkeit der innere Widerstand des Implantats in seinem expandierten Zustand gegen radial wirkende Kräfte verstanden, die eine radiale Kompression des Implantats bewirken können. Die Radialfestigkeit lässt sich dabei quantitativ durch Angabe eines Kollapsdruckes ausdrücken. Hierbei vollzieht sich bei Erreichen des Kollapsdruckes die Kompression schlagartig - das Implantat kollabiert.

In der Druckschrift EP 1 642 551 A1 wird ein Implantat beschrieben, das sich durch eine geringere Radialfestigkeit als davor bekannte Implantate auszeichnet. Es wurde nämlich festgestellt, dass eine geringere Radialfestigkeit gerade für biodegradierbare Implantate für eine Vielzahl von pathologischen Gefäßveränderungen nicht nur tolerabel ist, sondern zu einer deutlichen Verbesserung des Heilungsprozesses führt. In der Druckschrift EP 1 642 551 A1 wird daher ein Implantat vorgeschlagen, bei dem sich ausgehend vom expandierten Zustand die Querschnittsfläche bzw. das Innenvolumen mit steigendem radial angelegten Druck allmählich verringert, bis ein bestimmter, vorgegebener Druckwert überschritten wird. Das bekannte Implantat lässt sich daher bis zu einem festgelegten Grenzwert des Innenvolumens bzw. der Querschnittsfläche stetig komprimieren. Eine weitere Erhöhung des Kompressionsdrucks führt nicht zu einer weiteren Verringerung des Innenvolumens bzw. der Querschnittsfläche bis die Kompression den Kollapsdruck überschreitet. Bei weiterer Erhöhung des Kompressionsdrucks wird das Implantat kollabieren. Das Verhalten eines derartigen Implantats führt zu einer Verbesserung des Heilungsverlaufes, der jedoch noch immer nicht vollständig zufrieden stellend verläuft.

Es ist daher Aufgabe der vorliegenden Erfindung, eine Endoprothese zu schaffen, welche insbesondere hinsichtlich des Heilungsverlaufs und des Restenoseverhaltens noch weiter verbessert ist.

Die obige Aufgabe wird durch eine Endoprothese gelöst, welche eine vorzugsweise hohlzylinderförmige Grundstruktur aufweist, die hinsichtlich ihres Kompressionsverhaltens zwei Zustände einnimmt, nämlich einen Normalzustand und einen Zustand erheblicher Kompression. Erfindungsgemäß weist die Grundstruktur im Normalzustand eine Elastizität auf, durch die das durch die Grundstruktur eingeschlossene Innenvolumen elastisch reversibel veränderbar ist. Der Zustand erheblicher Kompression zeichnet sich dadurch aus, dass durch radial von außen wirkende Drücke der Innendurchmesser der Grundstruktur bzw. das verfügbare Innenvolumen signifikant verkleinert ist. Der Innendurchmesser liegt dabei unterhalb eines Durchmesser-Schwellwerts oder der Kompressionsdruck oberhalb eines Kompressionsdruck-Schwellwerts. Erfindungsgemäß ist im Zustand erheblicher Kompression die Elastizität der Grundstruktur signifikant gegenüber der Elastizität im Normalzustand verringert.

Bisher wurde eine elastische Rückfederung (Recoil) von Stents stets als Nachteil angesehen, da sie das verfügbare Lumen reduziert. Aus diesem Grund zielte das Design von Stents bisher, wie oben dargestellt, generell auf eine Minimierung dieses Recoil, so dass die Stents sehr starr ausgebildet waren. Außerdem werden Stents, ebenfalls um den Blutfluss nicht zu behindern, bei der Implantation generell auf den maximalen Durchmesser des Referenzgefäßabschnitts dimensioniert.

Bei dieser Ausgestaltung von Stents bleibt unberücksichtigt, dass ein gesundes Blutgefäß unter normalen physiologischen Bedingungen die weitaus meiste Zeit auf einem kleineren als seinem Maximaldurchmesser gehalten und es nur bei Bedarf an erhöhtem Blutfluss weiter gestellt wird. Dies bedeutet, dass das gesunde Blutgefäß eine gewisse Elastizität aufweist, die es im Normalzustand nutzt, um gewünschte Blutdurchfluss-Mengen zu realisieren.

Aufgrund des oben beschriebenen normalen physiologischen Verhaltens eines gesunden Blutgefäßes bedeutet die Implantation von starren Stents, die auf den maximalen Durchmesser des Gefäßes dimensioniert sind, eine fortwährende lokale Überdimensionierung des Gefäßes. Diese stellt einen bekannten Stimulus für die lokale Neointima-Bildung dar, welche eine Restenose induzieren oder verstärken kann.

Entgegen der etablierten Auffassung wird deshalb nun ein Designprinzip für Endoprothesen vorgeschlagen, bei dem im Normalzustand elastische Durchmesser-Änderungen im Rahmen der physiologisch normalen Variationen grundsätzlich erlaubt sind. Insbesondere wird durch die Erfindung das Missverhältnis zwischen dem durch die Endoprothese bereitgestellten Durchmesser und dem Durchmesser für den tatsächlich (im Mittel) benötigten Fluss der Körperflüssigkeit (z. B. Blut) beseitigt oder zumindest verringert. Hierbei sollen die bisher verwendeten Designprinzipien, nämlich, dass die Endoprothesen

- an der Gefäßwand anliegen und
- ein Volumen offen halten, das eine ausreichende Versorgung des Zielgebiets erlaubt,

beibehalten werden.

Erfindungsgemäß soll die Endoprothese ausgehend vom expandierten Zustand auf eine radiale Kompression wie folgt reagieren:

- Im Normalzustand wird eine elastische Rückfederung über einen großen Bereich (hierbei bedeutet groß, dass die Rückfederung der Größenordnung physiologischer Lumenvariationen, z. B. 20%, entspricht) zugelassen, wobei die zur Verformung nötigen Radialkräfte denen einer gesunden Gefäßmuskulatur entsprechen sollen.

- Im Zustand erheblicher Kompression soll ein Verhalten mit deutlich verringerter Elastizität bis hin zu einem starren Verhalten realisiert werden, sobald der Durchmesser zu einer potentiell flussbegrenzenden Stenose verkleinert wird. Hierbei bedeutet "starr", dass die Endoprothese eine Stützkraft aufweist, die einem Gefäßspasmus widerstehen kann.

Durch eine derartige erfindungsgemäße Endoprothese wird ein wesentlicher Reiz zur Neointimabildung wegfallen oder zumindest deutlich geschwächt werden. Mit einer derartigen Endoprothese behandelten Läsionen sind demnach weniger anfällig für eine Restenose.

Vorzugsweise ist die erfindungsgemäße Endoprothese so gestaltet, dass erst im Zustand erheblicher Kompression bei weiterer Erhöhung des Kompressionsdrucks bis zu einem Wert oberhalb des Kollapsdrucks ein irreversibles Versagen des Endoprothesen-Grundgitters auftritt.

In einem bevorzugten Ausführungsbeispiel wird die erfindungsgemäße Endoprothese durch eine Formgedächtnismetall-Legierung, beispielsweise Nitinol, realisiert. Eine solche Legierung zeichnet sich bereits inhärent durch einen großen elastischen Verformungsbereich aus. Endoprothesen, insbesondere Stents, aus einem selbst expandierenden Gedächtnismetall liegen zudem zuverlässig an der Wand des behandelten Gefäßes an.

Das im Wesentlichen gering elastische bis starre Verhalten im Zustand erheblicher Kompression sowie das erfindungsgemäße Verhalten im Normalzustand wird jedoch nicht allein durch die Materialwahl realisiert, sondern insbesondere durch die Gestaltung der Elemente der Grundstruktur der Endoprothese, welche nachfolgend anhand von einigen Beispielen dargestellt werden soll. Hierbei kann der Übergang der Elastizität vom Normalzustand in den Zustand erheblicher Kompression diskontinuierlich (z. B. hervorgerufen durch einen Anschlag oder eine Sperre) oder vorzugsweise auch kontinuierlich erfolgen (z.B. hervorgerufen durch eine Drehung von Hebelarmen).

Vorzugsweise besitzt die erfindungsgemäße Endoprothese ein sogenanntes Progressiv-Verhalten. Dies bedeutet, dass die Federkraft, d.h. die von der Grundstruktur bei einer Kompression gebildete rückwirkende oder rücktreibende Kraft überproportional mit dem Weg zunimmt. Dieses Verhalten wird insbesondere im Zustand erheblicher Kompression beobachtet. Grundsätzlich ist bei einer Kompression über den "inneren Anschlag" hinaus, d.h. im Zustand erheblicher Kompression, ein weiterhin elastisches Verhalten, lediglich mit einer dann stark erhöhten Federkonstante, erfindungsgemäß.

Im kräftefreien Zustand, der auch von dem Normalzustand umfasst wird, nimmt die erfindungsgemäße Endoprothese ihren Nominaldurchmesser ein. Dieser Durchmesser entspricht dem Durchmesser eines gesunden Referenz-Gefäßabschnitts bei maximaler medikamentöser Vasodilatation (z.B. durch NO). Das Verhalten der erfindungsgemäßen Endoprothese bei radialer Kompression im Normalzustand orientiert sich an den physiologischen Eigenschaften des jeweiligen Zielgefäßes. Häufig ist im Ruhezustand das Gefäß gegenüber dem Nominaldurchmesser um ca. 20% bis 25% enger gestellt.

Die Engstellung des Gefäßes wird durch eine Kontraktion in der Gefäßwand (Media) gegen den Widerstand des Blutdrucks bewirkt. Richtwerte für einen normalen arteriellen Blutdruck liegen im Bereich von etwa 130 mbar bis etwa 200 mbar (ca. 100 bis 150 mm Hg) systolisch bzw. kleiner als etwa 120 mbar (ca. 90 mm Hg) diastolisch. Im Normalzustand soll daher eine Reduzierung des Innendurchmessers der erfindungsgemäßen Endoprothese um 20% bis 25% gegenüber dem Nominaldurchmesser elastisch aufgrund von Radialkräften bewirkt werden können, wie sie von physiologischen Änderungen der Gefäßwandspannung hervorgerufen werden. Demzufolge soll sich der bevorzugte Druckbereich für den Normalzustand bis zu einem Kompressionsdruck-Schwellwert von etwa 200 mbar, vorzugsweise bis zu einem Kompressionsdruck-Schwellwert von 100 mbar, besonders bevorzugt bis zu einem Kompressionsdruck-Schwellwert von 75 mbar erstrecken. Oberhalb des genannten Kompressionsdruck-Schwellwerts befindet sich die erfindungsgemäße Endoprothese in dem Zustand erheblicher Kompression.

Bei einer weiteren Kompression der Endoprothese treten flussvermindernde Wirkungen in dem behandelten Gefäß ein, denen die erfindungsgemäße Endoprothese durch erhöhte Stützwirkung begegnen muss. Vorzugsweise ergibt sich ein sinnvoller Wert für das Ausmaß einer gegebenenfalls noch tolerablen weiteren elastischen Kompression aus dem minimal offenzuhaltenden Durchmesser, erhöht um einen Sicherheitszuschlag, der einer eventuell gebildeten Neointima und/oder einer nicht radialsymmetrischen Dilatation Rechnung trägt. In vielen Fällen kann die offenzuhaltende Durchmesserreduktion ca. 50% des Durchmessers betragen, wobei hierin ca. 25% Reserve einkalkuliert werden müssen. Folglich kann der Zustand erheblicher Kompression vorzugsweise dadurch charakterisiert werden, dass der Innendurchmesser auf 75% des Nominaldurchmessers reduziert ist. Dies ist etwa gleichbedeutend damit, dass der Kompressionsdruck den oben angegebenen Kompressionsdruck-Schwellwert überschreitet.

In einem bevorzugten Ausführungsbeispiel der erfindungsgemäßen Endoprothese beträgt ihr elastischer Widerstand im Zustand erheblicher Kompression mindestens das Zehnfache des elastischen Widerstands im Normalzustand. Diese Ausgestaltung der erfindungsgemäßen Endoprothese führt zu dem gewünschten starren Verhalten im Zustand erheblicher Kompression und kann, wie unten gezeigt wird, leicht beispielsweise durch eine entsprechende Anordnung von Stegen und Anpassung von Stegbreiten der Elemente der Grundstruktur realisiert werden. Der elastische Widerstand wird hierbei definiert als der extern anzulegende Druck in radialer Richtung bezogen auf eine durch diesen Druck hervorgerufene Durchmesseränderung.

Die erfindungsgemäße Endoprothese zeichnet sich in einem besonders bevorzugten Ausführungsbeispiel dadurch aus, dass im Zustand erheblicher Kompression in die Endoprothese eingeleiteten Kräfte überwiegend einen einachsigen Spannungszustand (Zugbeanspruchung oder Stauchung) von zumindest einem Teil der Elemente der Grundstruktur der Endoprothese bewirken. Mit einer solchen Ausgestaltung der erfindungsgemäßen Endoprothese lässt sich sehr einfach das gewünschte starre Verhalten der Endoprothese im Zustand erheblicher Kompression realisieren. Weiter bevorzugt bewirken die im Normalzustand in die Endoprothese eingeleiteten Kräfte überwiegend eine Biegebelastung von Elementen der Endoprothese, durch die das gewünschte elastische Verhalten der Endoprothese verwirklicht werden kann.

In einem weiteren Ausführungsbeispiel weist die Grundstruktur Mittel auf, die bei Unterschreiten des bestimmten, vorgegebenen Innendurchmesser-Schwellwerts aneinander anliegen.

In einem bevorzugten Ausführungsbeispiel sind die Mittel als eine Kerbe oder mehrere voneinander beabstandete Kerben (Schlitze) in Stegen der Grundstruktur ausgebildet, deren einander gegenüber liegende Wandabschnitte beim Unterschreiten eines bestimmten, vorgegebenen Krümmungsradius des jeweiligen Stegs im Zustand erheblicher Kompression derart aneinander anliegen, dass sie einer weiteren Verringerung des mittleren Krümmungsradius des jeweiligen Stegs entgegenwirken. Bei diesem Ausführungsbeispiel sind die Kerben an der Innenseite der Stege der Grundstruktur hinsichtlich der Krümmung angeordnet. Bei Kompression werden die Stege mit Kerbe(n) weiter gekrümmt.

In einer Abwandlung des zuvor genannten Ausführungsbeispiels sind die Mittel als eine Kerbe oder mehrere voneinander beabstandete Kerben (Schlitze) in Stegen der Grundstruktur ausgebildet, deren einander gegenüber liegende Wandabschnitte beim Überschreiten eines bestimmten, vorgegebenen Krümmungsradius des jeweiligen Stegs im Zustand erheblicher Kompression derart aneinander anliegen, dass sie einer weiteren Vergrößerung des mittleren Krümmungsradius des jeweiligen Stegs entgegenwirken. Bei diesem Ausführungsbeispiel sind die Kerben an der Außenseite der Stege der Grundstruktur hinsichtlich der Krümmung angeordnet. Bei dieser Abwandlung werden die Stege mit Kerbe(n) bei Kompression gerade gerichtet.

Bei beiden zuvor genannten Varianten eines Ausführungsbeispiels können die Kerbe oder die Kerben eine V-Form, eine U-Form, eine Rechteckform oder eine andere Form sowie eine kompliziertere, zusammengesetzte Form aufweisen. Bei der Form der Kerben kommt es weniger auf die Form des Bodens der Kerben an sondern vielmehr darauf, dass die Wände der Kerben in einem gewissen, vorgegebenen Abstand vom Boden im Zustand erhöhter Kompression in Kontakt gelangen.

Dieses Ausführungsbeispiel mit Kerbe oder Kerben beinhaltet eine einfache Realisierung von zwei Zuständen des Kompressionsverhaltens von Endoprothesen, eines mit einer großen Elastizität und eines mit einer vergleichsweise deutlich geringeren Elastizität. Im Normalzustand ist der effektive Durchmesser des jeweiligen Stegs durch die Kerbe oder Kerben deutlich verringert, so dass eine Verbiegung des Stegs leicht möglich ist und damit die gewünschte Elastizität erreicht wird. Der Zustand erheblicher Kompression wird durch einen "Anschlag" charakterisiert, bei dem einander gegenüber liegende Wandabschnitte der Kerben bzw. Schlitze aneinander anliegen. Nachdem die Seitenwandabschnitte aneinander anliegen, ist der effektive Durchmesser des jeweiligen Stegs um die Länge (Tiefe) der Kerbe erhöht und die Kraft, die zur Verformung benötigt wird, nimmt deutlich zu.

In einem weiteren Ausführungsbeispiel sind die Mittel als ein von dem jeweiligen Steg abstehendes, im Wesentlichen quer zur Längsrichtung der Endoprothese verlaufendes Anschlagelement, vorzugsweise als im Wesentlichen stabförmiger Stift oder Dorn, ausgebildet, wobei jedes Anschlagelement jeweils bei Unterschreiten des bestimmten, vorgegebenen Innendurchmesser-Schwellwerts an einem jeweils gegenüber liegenden Steg anliegt oder anschlägt. Hierfür sind die Enden der Anschlagelemente, die an dem gegenüber liegenden Steg anliegen, vorzugsweise verstärkt, insbesondere weisen sie einen größeren Durchmesser als der sich anschließende rückwärtige Abschnitt des Anschlagelements auf. In einem besonders bevorzugten Ausführungsbeispiel weist eine Vielzahl von Stegen jeweils ein derartiges Anschlagelement auf. Vorzugsweise hat der Stift oder Dorn einen rechteckigen Querschnitt, der sich einfach fertigen lässt.

Um ein definiertes Anschlagen bzw. ein definiertes Anliegen der Mittel im Zustand erheblicher Kompression zu gewährleisten bzw. ein seitliches Abgleiten der Anschlagelemente an dem gegenüber liegenden Steg zu verhindern, weisen die Mittel vorzugsweise Formschlussmittel auf, die im Zustand erheblicher Kompression einen Formschluss ausbilden.. Beispielsweise kann ein dem Anschlagelement gegenüber liegender Steg Kerben oder Vertiefungen aufweisen. Mit einer solchen Kerbe oder Vertiefung kann das entsprechende Ende des Anschlagelements in der Anschlagposition einen Formschluss ausbilden.

Die oben geschilderten Ausführungsbeispiele mit Kerben in den Stegen oder Anschlagelementen sind Beispiele dafür, dass im Zustand erheblicher Kompression in die Endoprothese eingeleitete Kräfte überwiegend eine Stauchung von Elementen der Grundstruktur bewirken. Bei den nachfolgend diskutierten Ausführungsbeispielen wird im Zustand erheblicher Kompression überwiegend eine Zugbeanspruchung von zumindest einem Teil der Elemente der Grundstruktur bewirkt.

Da es bei Implantationen in reale Stenosen, die häufig asymmetrisch oder partiell kalzifiziert sind, gegebenenfalls technisch schwierig ist, den Anschlag bei einer Kompression der Endoprothese zuverlässig zu realisieren oder diesen zuverlässig zu treffen, insbesondere wenn Distanzen von der Größenordnung der Maschengröße der Grundstruktur zu überbrücken sind, werden im Folgenden weitere Ausführungsbeispiele der vorliegenden Erfindung erläutert, die ohne Mittel zum Anliegen bzw. Anschläge auskommen.

Ein weiterer Nachteil von Lösungen, die einen Anschlag beinhalten, besteht darin, dass bei dem Aufeinanderpressen von zwei harten Teilen die Gefahr besteht, Gewebeteile bzw. Gefäßteile einzuklemmen. Hierbei können kleine Blutgefäße der Gefäßwand zur Versorgung der Gefäßwand (Vasa vasorum) oder weitere funktionswichtige Komponenten wie beispielsweise die Membrana elastica interna beschädigt werden. Verletzungen derartiger Komponenten können beispielsweise einen Entzündungsreiz, eine Einblutung oder eine Migrationsförderung bewirken.

In einem anschlagfreien Ausführungsbeispiel der erfindungsgemäßen Endoprothese weist die Grundstruktur im Wesentlichen quer zur Längsrichtung der Endoprothese verlaufende, im Normalzustand vorzugsweise gekrümmte Querstege sowie im Wesentlichen in Längsrichtung verlaufende Längsstege auf, wobei die Querstege und die Längsstege miteinander verbunden und in Umfangsrichtung der Endoprothese abwechselnd hintereinander angeordnet sind. Die Querrichtung und die Umfangsrichtung verlaufen senkrecht zur Längsrichtung der Endoprothese. Die Längsrichtung ist parallel zur Längsachse der Endoprothese.

Parallel zu einem Quersteg zwischen je zwei Verbindungsstellen ist jeweils ein Begrenzungssteg angeordnet, der, wenn der Krümmungsradius des Querstegs oberhalb eines bestimmten, vordefinierten Krümmungsradius-Schwellwerts des Querstegs liegt, d.h. im Zustand erheblicher Kompression, einer weiteren Vergrößerung des Krümmungsradius des Querstegs entgegenwirkt. Der Begrenzungssteg bildet somit im Zustand erheblicher Kompression eine Begrenzung für eine weitere Streckung des Querstegs und verhindert somit eine weitere Kompression der Endoprothese. Vorzugsweise ist der Begrenzungssteg im Zustand erheblicher Kompression gespannt. Bei Erreichen der maximalen Streckung jedes Querstegs werden demzufolge weitere Kompressionskräfte als Zugkräfte in den Begrenzungssteg eingeleitet, wodurch eine weitere Verbiegung (Krümmung) der Querstege verhindert wird. Bei diesem Ausführungsbeispiel sind die Längsstege vorzugsweise wesentlich steifer gegenüber einer Biegeverformung ausgebildet als die Querstege, so dass sich die Endoprothese im Zustand erheblicher Kompression durch eine Verformung der Längsstege nur mehr geringfügig weiter elastisch verformen kann. Das heißt, dass es einen deutlichen Unterschied im elastischen Verhalten zwischen den beiden Zuständen gibt. Der Begrenzungssteg weist vorzugsweise einen so kleinen Durchmesser auf, dass er im Normalzustand keinen wesentlichen Einfluss auf die Biegeverformung des jeweils parallelen Querstegs hat.

Bei einer weiteren anschlagfreien Ausführungsvariante weist die Grundstruktur Maschen auf, die durch jeweils zwei im Wesentlichen quer zur Längsrichtung der Endoprothese verlaufende Querstege ausgebildet sind, welche an ihren Enden miteinander verbunden sind, wobei beide Querstege (z.B. im Inneren einer Masche) zusätzlich mit mindestens einem im Wesentlichen in Längsrichtung der Endoprothese verlaufenden Konnektorsteg verbunden sind, der oberhalb eines bestimmten, vordefinierten Abstands der Querstege im Bereich des Konnektorstegs, d. h. im Zustand erheblicher Kompression, einer weiteren Vergrößerung des Abstands zwischen beiden Querstegen entgegenwirkt. Auch dieses Ausführungsbeispiel stellt eine einfache Möglichkeit dar, im Zustand erheblicher Kompression die von außen wirkenden Kompressionskräfte als Zugkräfte insbesondere in die in Längsrichtung verlaufenden Konnektorstege einzuleiten und somit die Elastizität der Grundstruktur zu begrenzen.

Es ist weiterhin bevorzugt, wenn die Grundstruktur durch abwechselnd quer zur Längsrichtung der Endoprothese hintereinander angeordnete und miteinander verbundene Querstege und Begrenzungsstege ausgebildet ist, wobei die Querstege im Normalzustand im Wesentlichen quer zur Längsrichtung und die Begrenzungsstege im Normalzustand zumindest abschnittsweise im Wesentlichen in Längsrichtung der Endoprothese verlaufen, und wobei weiterhin die Begrenzungsstege mit zunehmender Kompression derart drehbar sind, dass sie zunehmend quer zur Längsrichtung verlaufen, wobei die Elastizität der Begrenzungsstege bei zunehmender Verdrehung abnimmt, wobei im Normalzustand die Federkonstante a der Querstege größer ist als die Federkonstante b der Begrenzungsstege, vorzugsweise a > 10 ·b, und im Zustand erheblicher Kompression die Zugfestigkeit der Begrenzungsstege größer ist als die zur Verbiegung der Querstege nötige Kraft. Auch bei diesem Ausführungsbeispiel wird durch einfache Mittel erreicht, dass die eingeleiteten Kräfte mit zunehmender Kompression zu einem immer größer werdenden Anteil als Zugkräfte von den Begrenzungsstegen aufgenommen werden, wobei die Begrenzungsstege zunehmend in die Richtung quer zur Längsrichtung drehen. Nach dem Strecken der Begrenzungsstege kann eine weitere Elastizität nur noch durch eine Verbiegung der Querstege erreicht werden, die jedoch vergleichsweise steif gegenüber einer Verbiegung ausgebildet sind.

Bei den oben genanten anschlagfreien Ausführungsbeispielen ist es von Vorteil, wenn die Begrenzungsstege oder die Konnektorstege eine kleinere Breite als die Querstege aufweisen, wobei die Breite der Begrenzungsstege oder der Konnektorstege vorzugsweise kleiner als 1/3 der Breite der Querstege ist. Hierdurch wird gewährleistet, dass die Konnektorstege bzw. Begrenzungsstege im Normalzustand keinen oder nur einen sehr geringen Einfluss auf das elastische Verhalten der Endoprothese haben.

Es ist weiter von Vorteil, wenn die Grundstruktur in einem gecrimpten Zustand einen Innendurchmesser aufweist, der signifikant kleiner ist als der Innendurchmesser der Endoprothese im Zustand erheblicher Kompression. Hierdurch ist es einfach möglich, die Endoprothese in das zu behandelnde Körperteil einzubringen und dort zu dilatieren. Diese Behandlungsmethode hat sich bewährt, da sie minimalinvasiv durchgeführt werden kann und kostengünstig ist.

Weitere Ziele, Merkmale, Vorteile und Anwendungsmöglichkeiten der vorliegenden Erfindung ergeben sich auch aus der nachfolgenden Beschreibung von Ausführungsbeispielen anhand von Figuren. Dabei bilden alle beschriebenen und/oder bildlich dargestellten Merkmale für sich oder in beliebiger Kombination den Gegenstand der Erfindung, auch unabhängig von ihrer Zusammenfassung in einzelnen Ansprüchen oder deren Rückbeziehung.

Die nachfolgend aufgeführten Figuren (außer die Figuren 9 bis 11) verstehen sich als Ansicht von der Seite auf einen Ausschnitt der Grundstruktur des Stents. Es zeigen schematisch:

Figur 1        einen Abschnitt eines Stegs eines ersten Ausführungsbeispiels einer erfindungsgemäßen Endoprothese,

Figur 2        einen Abschnitt eines Stegs eines zweiten Ausführungsbeispiels einer erfindungsgemäßen Endoprothese,

Figur 3        einen Abschnitt eines Stegs eines dritten Ausführungsbeispiels einer erfindungsgemäßen Endoprothese,

Figur 4        einen Abschnitt der Grundstruktur eines vierten Ausführungsbeispiels einer erfindungsgemäßen Endoprothese,

Figur 5        zwei weitere Varianten der Ausgestaltung eines Stegs zu dem vierten Ausführungsbeispiel einer erfindungsgemäßen Endoprothese,

Figur 6        einen Abschnitt der Grundstruktur eines fünften Ausführungsbeispiels einer erfindungsgemäßen Endoprothese in einer Ansicht von der Seite,

Figur 7        einen Abschnitt der Grundstruktur eines sechsten Ausführungsbeispiels einer erfindungsgemäßen Endoprothese,

Figur 8        einen Abschnitt der Grundstruktur eines siebten Ausführungsbeispiels einer erfindungsgemäßen Endoprothese,

Figuren 9 und 10     den Verlauf von Kräften, die an Stegen anliegen, und

Figur 11      die elastische Dehnung eines Stegs im gecrimpten Zustand gegenüber der Dehnung im Normalzustand.

Eine als Stent ausgebildete Endoprothese ist aus einer Gitterstruktur von in Längsrichtung 5 und in Querrichtung verlaufenden Stegen zusammengesetzt, die einen hohlzylindrischen Gitterkörper ausbilden. Die nachfolgend anhand der Figuren erläuterten Stents weisen jeweils eine derartige Gitterstruktur auf, wobei jeweils die Querrichtung bzw. Umfangsrichtung senkrecht zur Längsrichtung 5 (Richtung der Achse des Hohlzylinders) verläuft.

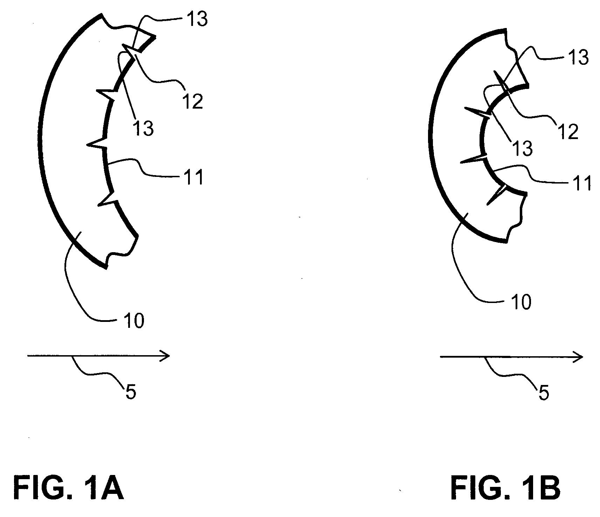

In Figur 1 ist ein im Wesentlichen in Querrichtung verlaufender Steg 10 eines Stents gezeigt, der im Normalzustand (siehe Figur 1a) leicht gekrümmt ist. Auf der Innenseite 11 eines solchen Stegs, die den kleinsten Biegeradius aufweist, sind V-förmige Kerben 12 in den Steg 10 eingebracht, welche im Normalzustand die effektive Stegbreite verringern und so eine große Elastizität des Stegs 10 bei der Einwirkung äußerer Kompressionskräfte bewirken. Die Kompressionskräfte wirken dabei in der Regel in radialer Richtung auf den Stent ein.

Bei einer Kompression (siehe Figur 1b) wird der Biegeradius des Stegs 10 verringert. Hierdurch werden die V-förmigen Kerben auf der Innenseite 11 zunehmend verschlossen und die einander gegenüberliegenden Seitenwände 13 der Kerben 12 nähern sich so weit aneinander an, bis sie schließlich aneinander anliegen. Deshalb wird im Zustand erheblicher Kompression, nämlich dann, wenn ein vorgegebener Schwellwert für den Biegeradius der Innenseite 11 des Stegs unterschritten wird, eine größere effektive Stegbreite als im Normalzustand realisiert. Der Unterschied zwischen den effektiven Stegbreiten im Normalzustand und im Zustand erheblicher Kompression ist durch die Tiefe der Kerben gegeben. Durch die erhöhte effektive Stegbreite wird einer äußeren Kompressionskraft ein größerer elastischer Widerstand entgegengesetzt. Im Zustand erheblicher Kompression ist daher die Elastizität des Stegs gegenüber dem Normalzustand deutlich verringert.

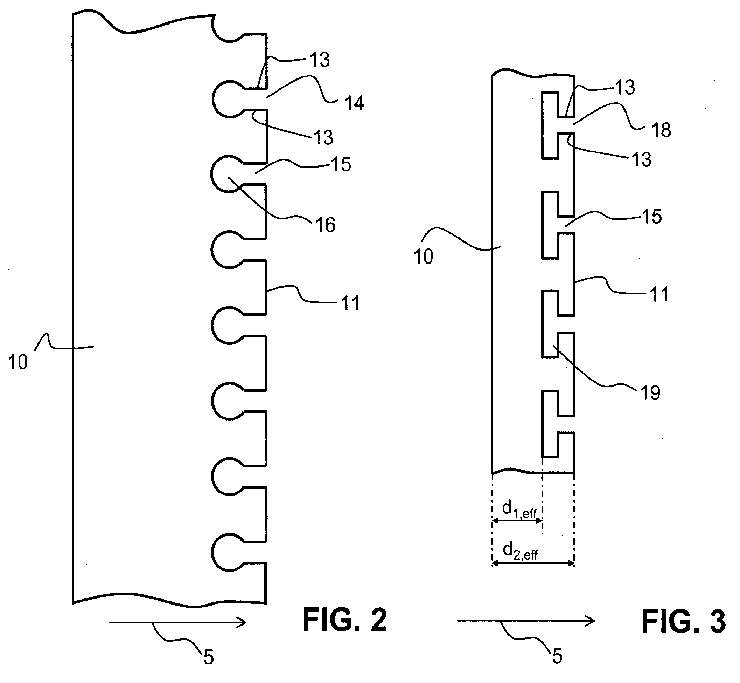

Die Figuren 2 und 3 zeigen weitere Ausführungsbeispiele, die sich von der Figur 1 dargestellten Ausführungsform in der Form ihrer Kerben (Schlitze) unterscheiden.

Bei dem in Figur 2 dargestellten Ausführungsbeispiel sind Kerben 14 vorgesehen, welche sich jeweils aus einem Halsabschnitt 15 mit geraden Seitenwänden und einem inneren Abschnitt 16 mit Zylindermantel-förmigen Seitenwänden zusammensetzen. Der zylinderförmige Abschnitt 16 ist jeweils weiter im Inneren des jeweiligen Stegs 10 angeordnet als der Halsabschnitt 15.

In dem in Figur 3 dargestellten Ausführungsbeispiel ist der zylinderförmige Abschnitt 16 der in Figur 2 gezeigten Kerben 14 ersetzt durch einen quaderförmigen Abschnitt 19. Der quaderförmige Abschnitt 19 schließt sich bei der Kerbe 18 unmittelbar an den jeweiligen Halsabschnitt 15 an.

Die in den Figuren 2 und 3 dargestellten Ausführungsbeispiele können auch gemäß ihrer Form als "Drachenrücken"- oder "dragon back"-Kerben bezeichnet werden. In Figur 3 sind die effektiven Durchmesser für die Verbiegung eines Stegs 10 eingezeichnet. Im Normalzustand wirkt der effektive Durchmesser $d_{1,eff}$, der sich von der der Kerbe 18 gegenüberliegenden Seite des Stegs 10 bis zu dem vorderen, am weitesten im Inneren des Stegs 10 liegenden Ende der Kerbe 18 erstreckt. In dem Zustand erheblicher Kompression wirkt der Durchmesser $d_{2,eff}$, der dem Gesamtdurchmesser des Stegs 10 entspricht.

Die in den Figuren 2 und 3 dargestellten Ausführungsbeispiele einer erfindungsgemäßen Endoprothese lassen sich praktisch besser realisieren als das in Figur 1 dargestellte Ausführungsbeispiel. Hierbei können durch geeignete Wahl der geometrischen Dimensionen unabhängig voneinander das geforderte Verhältnis der Federkonstanten des Normalzustands bezogen auf den Zustand erheblicher Kompression (nämlich beispielsweise durch geeignete Wahl des Verhältnisses $d_{1,eff}$ / $d_{2,eff}$) und der geforderte Verformungsweg zwischen dem kräftefreien Normalzustand bis zum Zustand erheblicher Kompression (nämlich beispielsweise durch geeignete Wahl des Verhältnisses d(Halsabschnitt) / $d_{2,eff}$) eingestellt werden. Hierbei bedeutet d(Halsabschnitt) die Breite der Kerbe im Normalzustand im Bereich des jeweiligen Halsabschnitts 15.

Die in den Figuren 1 bis 3 dargestellten Kerben können in die Stege vorzugsweise mittels Laserschneiden eingebracht werden.

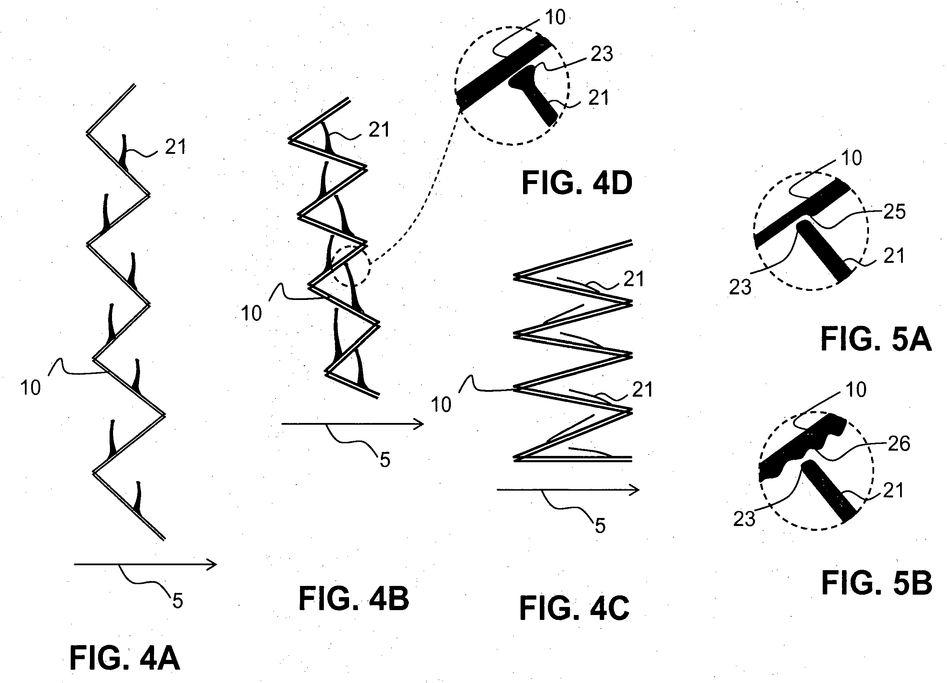

In den nachfolgend beschriebenen Figuren 4 bis 8 sind Grundstruktur-Segmente aufgeschnitten gezeigt, welche entlang der Umfangsrichtung (quer zur Längsrichtung 5) verlaufen und den vollen Umfang des Stents oder einem Teil davon bilden können. Ferner können die dargestellten Segmente kreisförmig oder helixförmig angeordnet sein.

Bei dem in Figur 4 dargestellten vierten Ausführungsbeispiel ist das Grundstruktur-Segment aus Stegen 10 zusammengesetzt, welche in einer Zickzack-Struktur angeordnet sind. Benachbart zu dem dargestellten Segment sind weitere solcher Segmente angeordnet. An einer großen Anzahl von Stegen 10 sind erfindungsgemäß im Wesentlichen stabförmige Anschlagelemente 21 in Form von Stiften vorgesehen, welche vorzugsweise unter einem bestimmten, fest vorgegebenen Winkel von dem jeweiligen Steg 10 abragen. In Figur 4a ist eine Situation dargestellt, in der der Stent im Normalzustand vorliegt. In diesem Zustand kann der Stent beispielsweise einen Innendurchmesser von 4 mm aufweisen.

Im Normalzustand ist die Grundstruktur des Stents in weiten Grenzen elastisch verformbar. Beispielsweise ist die elastische Verformung durch S-förmige Krümmung der einzelnen miteinander verbundenen Stege 10 möglich. Eine Kompression durch Radialkräfte führt dazu, dass sich der Stentdurchmesser verringert, so dass sich die benachbarten Stege 10 aufeinander zu bewegen.

Eine solche Bewegung und damit eine elastische Kompression ist so lange möglich, bis die von den Stegen abragenden Anschlagelemente 21, wie in Figur 4b dargestellt, an den jeweils gegenüber liegenden Steg 10 anstoßen. Eine vergrößerte Darstellung der Grundstruktur in dem Bereich, in dem ein Anschlagelement 21 an einen gegenüber liegenden Steg 10 anstößt, ist in Figur 4d gezeigt. In dieser Zeichnung ist auch erkennbar, dass in dem dargestellten bevorzugten Ausführungsbeispiel das Ende 23 des Anschlagelements 21 einen gegenüber dem rückwärtigen Abschnitt des Anschlagelements 21 größeren Durchmesser aufweist. Hierdurch wird die Kontaktfläche zwischen gegenüber liegendem Steg 10 und Anschlagelement 21 beim Anschlagen vergrößert, so dass ein sichererer Kontakt zwischen Anschlagelement 21 und Steg 10 ausgebildet wird. In dieser Ausgestaltung ist es vorteilhaft, wenn der zwischen dem Anschlagelement 21 und dem Steg 10 ausgebildete Winkel bei 90° liegt, so dass das Anschlagelement 21 bei Kompression nicht seitlich weggedrückt wird. Bei Abweichung von 90° gibt es nämlich beim Andrücken immer eine Querkomponente der Kraft, die das Anschlagelement 21 seitlich wegrutschen lässt.

In dem in Figur 4b gezeigten Zustand erheblicher Kompression wird eine weitere elastische Verformung der Grundstruktur durch die Anschlagelemente 21 begrenzt, die eine weitere Verringerung des Abstands zwischen benachbarten Stegen 10 bzw. eine Verkleinerung des zwischen benachbarten Stegen 10 eingeschlossenen Winkels verhindern. Bei weiterer Erhöhung der auf die Grundstruktur wirkenden Kompressionskräfte werden diese durch die Anschlagelemente 21 aufgenommen und führen zu einer Stauchung oder Verbiegung der Anschlagelemente 21. Da sich durch zusätzliche Wirkung der Anschlagelemente 21 die Elastizitätskonstante gegenüber der Elastizitätskonstante der allein aus den Stegen 10 und deren Verbindungsstellen gebildeten Struktur erhöht, ist die elastische Verformung der Grundstruktur im Zustand erheblicher Kompression geringer.

Im Zustand erheblicher Kompression kann der Stentdurchmesser beispielsweise etwa 3 mm betragen.

Figur 4c stellt den Zustand dar, in dem der Stent beispielsweise auf einen Katheter aufgecrimpt ist. In diesem Zustand ist der Stent noch nicht dilatiert. In einem solchen Zustand kann der Stent beispielsweise etwa einen Durchmesser von 1,4 mm aufweisen. Im gecrimpten Zustand liegen die Anschlagelemente 21 zumindest mit einem Teil ihrer Länge an dem jeweiligen Steg 10 an, von dem sie im Normalzustand abragen. Bei einer Verringerung der Abstände zwischen benachbarten Stegen 10 kann demnach das Anschlagelement 21 nicht mehr einer weiteren Komprimierung entgegenwirken.

Anhand von Figur 5 sind zwei weitere Möglichkeiten dargestellt, wie der Anschlag des Anschlagelements 21 an dem gegenüber liegenden Steg 10 realisiert werden kann. Ziel dieser Möglichkeiten ist es, die Toleranz gegenüber Variationen in der genauen Orientierung des Anschlagelements 21 zum Steg 10 zu erhöhen, wie sie aufgrund von Fertigungstoleranzen und aufgrund des Einflusses der Umgebung am Implantationsort zu erwarten sein werden.

In dem in Figur 5a dargestellten Beispiel weist der Steg 10 im Anschlagbereich einen Absatz 25 bzw. eine Vertiefung 25 auf, an dem das Anschlagelement 21 im Zustand erheblicher Kompression formschlüssig anliegt. Der Absatz 25 ist dabei derart gestaltet, dass er eine Verschiebung des Anschlagelements 21 nach außen, d.h. in die der Verbindung der benachbarten Stege 10 gegenüber liegende Richtung, verhindert. Bei diesem Ausführungsbeispiel ist es vorteilhaft, wenn der Winkel zwischen dem Anschlagelement 21 und dem Steg 10 kleiner als 90° ist, so dass das Anschlagelement 21 bei Kompression in den Absatz 25 hineingedrückt wird. Hierbei kann ein gewisser Winkelbereich unterhalb von 90° toleriert werden; maximal soweit, wie es die maximale Neigung der Kante des Absatzes 25 vorgibt. Bei einem Winkel, der zu weit unter 90° liegt, würde das Anschlagelement 21 über den Absatz 25 rutschen; bei einem Winkel, der deutlich über 90° liegt, könnte es ungehindert von dem Absatz 25 weg abgleiten.

Das in Figur 5b dargestellte Ausführungsbeispiel stellt einen Steg mit einer an der Oberfläche ausgebildeten wellenförmigen Struktur dar, in deren Vertiefungen 26 das Anschlagelement 21 im Zustand erheblicher Kompression formschlüssig eingreifen kann. Eine derartige wellenförmige Struktur verhindert die Verschiebung des Anschlagelements 21 nach außen und innen. In dieser Ausgestaltung ist es vorteilhaft, wenn der Winkel zwischen dem Anschlagelement 21 und dem Steg 10 etwa bei 90° liegt, so dass das Anschlagelement 21 bei Kompression in die getroffene Vertiefung 26 hineingedrückt wird. Hierbei können auch gewisse Abweichungen von 90° toleriert werden; maximal soweit, wie es die maximale Neigung der Vertiefungen 26 vorgibt. Erst bei größeren Abweichungen würde das Anschlagelement dann seitlich aus der Mulde rutschen.

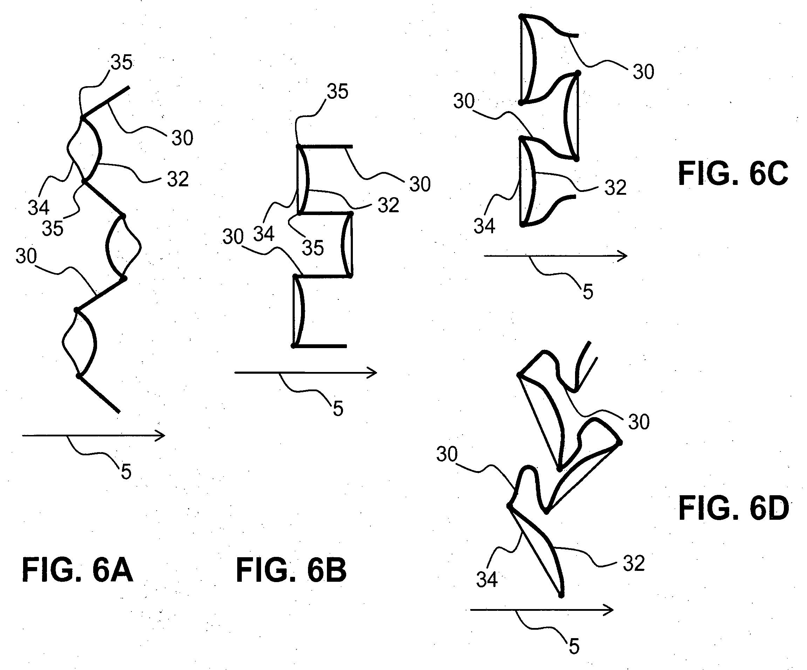

Figur 6 zeigt ein Ausführungsbeispiel eines Stents, dessen Grundstruktur sich aus im Wesentlichen in Längsrichtung 5 verlaufenden Längsstegen 30 und im Wesentlichen in Umfangsrichtung (d.h. senkrecht zur Längsrichtung 5) verlaufenden Querstegen 32 zusammensetzt. Jeweils ein Längssteg 30 ist mit einem Quersteg 32 in Querrichtung abwechselnd nacheinander angeordnet. Parallel zu jedem Quersteg 32 ist ein Begrenzungssteg 34 angeordnet, der an seinen Enden mit je einem Endpunkt 35 des jeweiligen Querstegs 32 verbunden ist.

Figur 6a zeigt ein Segment der Grundstruktur im Normalzustand. Die Elastizität der Grundstruktur wird durch die Elastizität der Querstege 32 und der Längsstege 30 bestimmt, wobei vorzugsweise die Verbindungsstellen 35 zwischen einem Quersteg 32 und einem Längssteg 30 winkelstabil, d.h. massiv, ausgebildet sind. Bei einer Kompression des erfindungsgemäßen Stents werden zunächst vor allem die Querstege 32, die eine kleinere Elastizitätskonstante aufweisen, so verbogen, dass ihr Krümmungsradius erhöht wird. Dies führt dazu, dass sich der Abstand der Endpunkte 35 der Querstege 32 vergrößert und jeder Begrenzungssteg 34 mit zunehmender Kompression zunehmend gerader bzw. gestreckter verläuft, bis der in Figur 6b gezeigte Zustand erheblicher Kompression vorliegt. In diesem Zustand entspricht der Abstand der Endpunkte 35 eines Querstegs 32 der Länge des jeweils parallelen Begrenzungsstegs 34 und der Begrenzungssteg 34 ist gespannt. In diesem Zustand werden weitere Kompressionskräfte durch die Begrenzungsstege 34 als Zugkräfte aufgenommen, so dass die Elastizität der Grundstruktur hierdurch begrenzt wird. Ein Teil der Kompressionskräfte kann auch zu einer Verbiegung der Längsstege 30 führen, wobei die Längsstege 30 vorzugsweise deutlich steifer als die Querstege 32 ausgebildet sind.

Vorzugsweise ist die Federkonstante C der Begrenzungsstege 34 kleiner als die Federkonstante B des Querstegs 32 und diese wiederum kleiner als die Federkonstante A des Längsstegs 30 (C < B < A, vorzugsweise C « B « A), wobei alle genannten Federkonstanten Federkonstanten gegen Verbiegung darstellen. In einem weiteren Ausführungsbeispiel kann der Längssteg 30 auch S-förmig ausgebildet sein. Ferner muss der von einem Längssteg 30 und einem Quersteg 32 im Verbindungspunkt 35 eingeschlossene Winkel nicht unbedingt 90° betragen, sondern kann etwas kleiner als ein rechter Winkel bzw. auch deutlich größer als 90° ausgebildet sein.

Im Zustand erheblicher Kompression wird somit eine größere Elastizität durch die Streckung des Begrenzungsstegs 34 verhindert, so dass das Verhalten des Stents bei einer weiteren Kompression als hart charakterisiert werden kann. Ein weiteres Federn erfolgt lediglich durch Verbiegen der Längsstege 30, die jedoch eine große Federkonstante aufweisen.

Anhand der Figuren 6c und 6d werden zwei Beispiele dargestellt, die ein Segment der Grundstruktur im gecrimpten Zustand zeigen. Es wird deutlich, dass im gecrimpten Zustand der Längssteg 30 S-förmig ausgebildet ist, so dass ein kleiner Innendurchmesser der Grundstruktur des erfindungsgemäßen Stents realisiert werden kann.

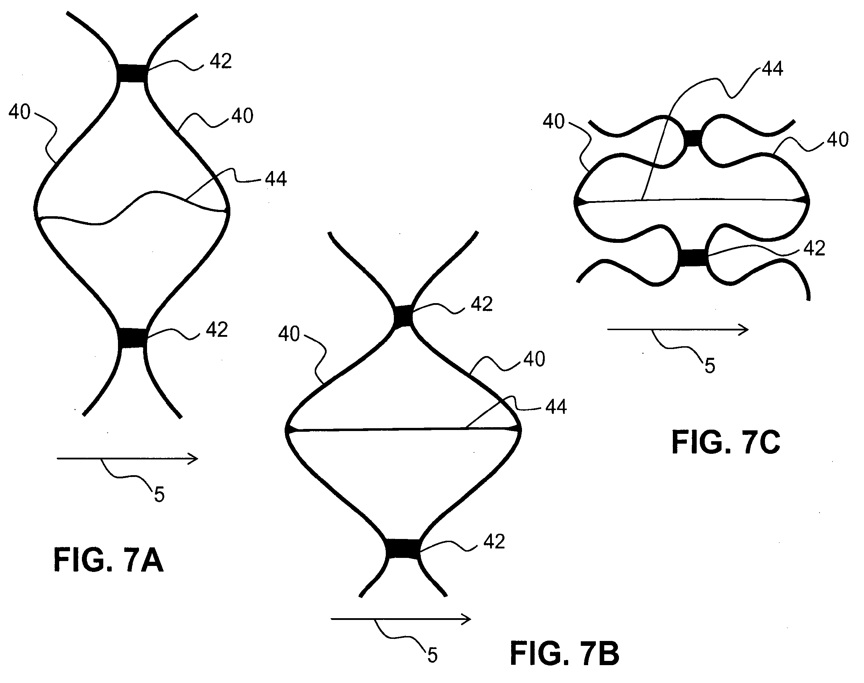

Bei dem in Figur 7 dargestellten Ausführungsbeispiel setzt sich die Grundstruktur eines erfindungsgemäßen Stents aus Maschen zusammen, die jeweils durch zwei im Wesentlichen in Quer- oder Umfangsrichtung verlaufende Querstege 40 ausgebildet werden. Die Querstege 40 sind jeweils an ihren Enden durch jeweils eine Anschlussstelle 42 miteinander verbunden. Etwa im mittleren Bereich jedes Querstegs 40 ist ein Konnektorsteg 44 angeordnet, welcher bei erhöhter Kompression eine weitere Verbreiterung der jeweiligen Masche und damit eine weitere Reduzierung des Innendurchmessers des Stents verhindert.

Figur 7a zeigt den Normalzustand, in dem die Elastizität der Grundstruktur durch die Elastizität der Stege 40 gegeben ist. Überschreitet bei weiterer Kompression des Stents der Abstand der beiden Querstege 40 einen bestimmten, vorgegebenen Schwellwert, dann ist der Konnektorsteg 44 gespannt bzw. gestreckt. Dieser Zustand erheblicher Kompression ist in Figur 7b dargestellt. Bei weiterer Kompression des Stents setzen die gespannten Konnektorstege 44 den aufgebrachten Kräften, die als Zugkräfte in die Konnektorstege 44 eingeleitet werden, einen Widerstand entgegen, so dass die Elastizität der Grundstruktur in diesem Zustand deutlich sinkt.

In dem in Figur 7c dargestellten Zustand ist der Stent auf einen Katheter gecrimpt, wobei in diesem Zustand die oberhalb und unterhalb zu jedem Konnektorsteg 44 angeordneten Abschnitte jedes Querstegs 40 S-förmig verbogen sind. Durch die in Figur 7c angegebene Verformung der Querstege 40 kann der Stent im gecrimpten Zustand einen deutlich kleineren Durchmesser einnehmen als in dem in Figur 7b dargestellten Zustand erheblicher Kompression.

Abschließend soll noch darauf hingewiesen werden, dass die Anschlussstellen 42 des in Figur 7 dargestellten Stents winkelstabil bzw. steif ausgeführt sind.

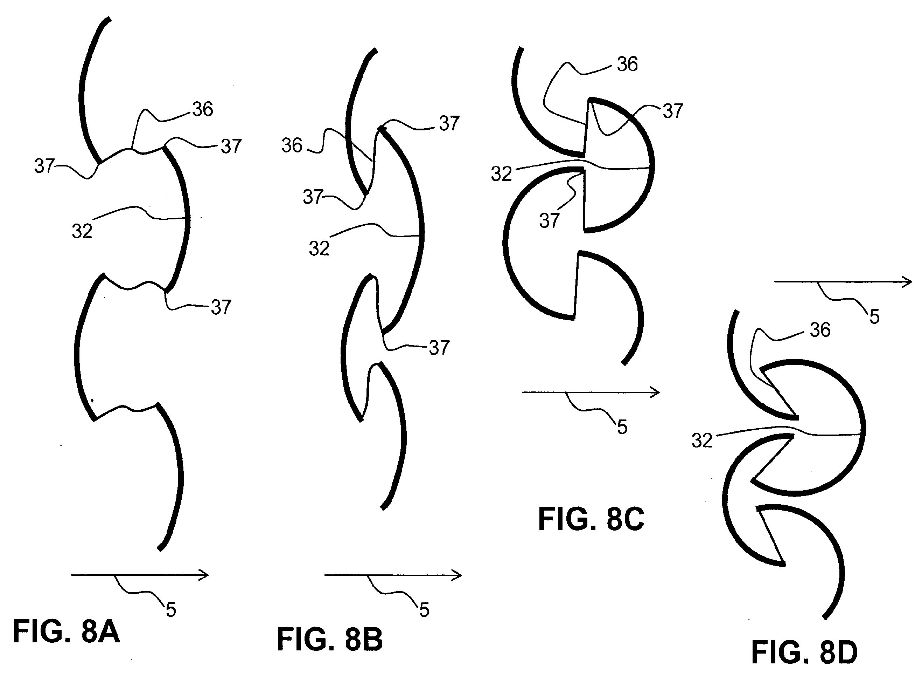

Figur 8 zeigt ein weiteres anschlagfreies Ausführungsbeispiel eines Stents, bei dem sich die Grundstruktur aus im Wesentlichen in Querrichtung verlaufenden Querstegen 32 und im Wesentlichen in Längsrichtung verlaufenden Längsstegen 36 zusammensetzt, die jeweils in Querrichtung bzw. Umfangsrichtung abwechselnd nacheinander angeordnet sind.

Figur 8a zeigt wiederum den Normalzustand. Die Querstege 32 sind vergleichsweise starr ausgebildet, während die Grundstruktur durch eine Verbiegung der Längsstege 36 federn kann. Die Verbiegung geht mit einer allmählichen Drehung der Längsstege 36 in die Umfangsrichtung einher, die in der Ebene des Umfangs des Stents erfolgt. Bei einer erhöhten Kompression wird der Durchmesser des Stents durch die Drehung der Längsstege 36 verringert bis zu dem Zustand erheblicher Kompression, der in Figur 8b dargestellt ist. Dieser Zustand kann als hart charakterisiert werden, da die Elastizität des Hebelarms (Längssteg) 36 mit Vergrößerung des Drehwinkels abnimmt. Je mehr also der Hebelarm 36 in Quer- bzw. Umfangsrichtung dreht, desto weniger kann er noch federn. Nachdem die Drehung des Längsstegs 36 soweit vollzogen ist, dass dieser weitgehend gestreckt in Querrichtung verläuft, kann ein weiteres Federn nur noch durch Verbiegen der Querstege 32 erfolgen, die jedoch eine deutlich größere Federkonstante aufweisen als die Längsstege 36.

In den Figuren 8c und 8d sind zwei mögliche Ausführungsformen des in Figur 8 dargestellten Stents im gecrimpten Zustand gezeigt, in dem der Stent einen noch kleineren Durchmesser als im Zustand erheblicher Kompression aufweist. Die gezeigten Zustände werden durch eine so erhebliche Biegung der Querstege 32 erreicht, dass diese im Wesentlichen kreisförmig ausgebildet sind. Hierbei ist zu beachten, dass die Länge des Längsstegs 36 kleiner ist als (Länge des Quersteges 32)/$\pi$, da sonst die in den Figuren 8c und 8d gezeigten gecrimpten Zustände nicht realisiert werden können.

Aus obigen Ausführungsbeispielen, insbesondere aus dem anhand von Figur 8 erläuterten Ausführungsbeispiel geht ein allgemeines Prinzip der vorliegenden erfindungsgemäßen Endoprothese hervor. Die Grundstruktur ist vorzugsweise so ausgebildet, dass zwei Konstruktionselemente mit unterschiedlicher Biegesteifigkeit kombiniert werden, und zwar derart, dass bei kleinen Belastungen im Normalzustand vorwiegend das weichere Element (z.B. in dem in Figur 8 dargestellten Ausführungsbeispiel der Längssteg 36) und bei höheren Belastungen im Zustand erheblicher Kompression das härtere Element (z.B. in Figur 8 der Quersteg 32) beansprucht wird.

Der Übergang zwischen beiden Lastbereichen erfolgt beispielsweise dadurch, dass das weichere Element bei leichter Kompression zunächst gebogen wird, bei fortschreitender Kompression aber zunehmend in die Umfangsrichtung oder Querrichtung gedreht wird, so dass eine Biegebelastung im Normalzustand kontinuierlich oder diskontinuierlich im Zustand erheblicher Kompression in einen überwiegenden einachsigen Spannungszustand (Zugbelastung oder Stauchung) übergeht. Ein solcher einachsiger Spannungszustand führt zu einem "härteren" Verhalten als eine Biegebelastung.

Um das erfindungsgemäße Verhalten zeigen zu können, ist vorzugsweise die Belastbarkeit des jeweils unter Zugbeanspruchung stehenden Elements der Grundstruktur bei Zugbeanspruchung mindestens 10 Mal so hoch wie bei einer Biegebeanspruchung.

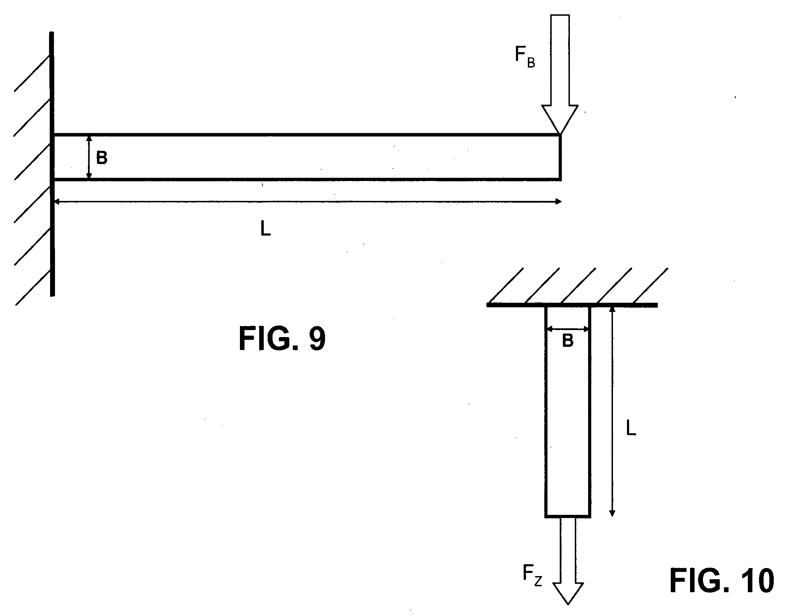

Anhand der in den Figuren 9 und 10 gezeigten Schemazeichnungen wird im Folgenden dargestellt, dass die in dem vorangegangenen Abschnitt formulierte Forderung leicht zu erreichen ist. Bei der in Figur 9 gezeigten reinen Biegung eines als Balken dargestellten Stegs wirkt eine Kraft $F_B$ quer zur Achse des Balkens der Länge L und der Breite B sowie der Dicke D. Hierbei ist die Dicke D in Figur 9 nicht eingezeichnet. Diese Kraft bewirkt ein Drehmoment $M = F_B \cdot L$, das den Balken krümmt, wobei die stärkste Krümmung an der Einspannstelle auftritt (maximales Drehmoment beim maximalen Abstand vom Angriffspunkt der Kraft). Unter der Annahme, dass sich beim Biegen die relative Dehnung und Stauchung $\varepsilon$ linear und symmetrisch um eine "neutrale Faser" in der Mitte des Balkens verteilen, gilt für das Gegen-Drehmoment durch den Balken

$$M = \int dy \int dz\, E \cdot \varepsilon\ (y,z) \cdot y = \int dy \int dz\, E \cdot (\varepsilon_{max} \cdot y/(B/2)\,) \cdot y = E \cdot \varepsilon_{max} \cdot B^2/6 \cdot D,$$

bzw. für die zulässige Kraft

$$F_B = M_{max} / L = E \cdot \varepsilon_{max} \cdot B^2/6 \cdot D / L = \varepsilon_{max} \cdot E \cdot B \cdot D \cdot (B / 6L).$$

Hierbei bezeichnen E den Elastizitätsmodul und $\varepsilon_{max}$ die maximale elastische Dehnung des Werkstoffs, sowie x / y / z die Richtungen entlang der Länge L / der Breite B / der Dicke D des unbelasteten Balkens.

Eine reine Zugbelastung eines als Balken vereinfachten Stegs wird in Figur 10 dargestellt. Eine Kraft $F_Z$ wirkt längs der Achse eines Balkens der Länge L, der Breite B und der Dicke D. Aus der vorgegebenen Zugspannung $\sigma = F_Z / B \cdot D$ und dem Elastizitätsmodul E des Werkstoffs kann die zugehörige relative Dehnung $\varepsilon = \Delta L / L$ aus $\sigma = \varepsilon \cdot E$ berechnet werden. Hieraus ergibt sich für die zulässige Kraft

$$F_Z = \sigma_{max} \cdot B \cdot D = \varepsilon_{max} \cdot E \cdot B \cdot D.$$

Somit unterscheidet sich die maximale Zugkraft $F_Z$ um den Faktor 6L/B von der zulässigen Biegekraft $F_B$. Da für Geometrien, wie sie für Stege von Stents verwendet werden, praktisch in allen Fällen gilt L >> B, bedeutet die Forderung $F_Z > 10 \cdot F_B$ in allen praktischen Fällen keine Einschränkung für das Design, sondern kann leicht realisiert werden.

Folglich kann sehr leicht beispielsweise bei der in Figur 8 dargestellten Grundstruktur realisiert werden, dass die Querstrebe 32 steifer ist als die Längsstrebe 36. Dieses kann z.B. sehr effektiv durch eine Anpassung der Stegbreite B erreicht werden, die quadratisch in die Biegekraft eingeht (siehe Rechnung zu Figur 9). Soll beispielsweise (bei gleicher Steglänge) eine zehnfache Kraft für eine Biegung des Querstegs 32 wie für die Biegung des Längsstegs 36 realisiert werden, so ist die Breite des Querstegs 32 so zu dimensionieren, dass sie $\sqrt{10}$ - fach, also rund 3,2-fach, größer ist als die Breite des Längsstegs 36. Die übrigen Abmessungen der Stege 32, 36 sind gleich. Eine derartige Variation der Stegbreite lässt sich beispielsweise mit den derzeit vorhandenen Methoden der Laserstrukturierung problemlos fertigen.

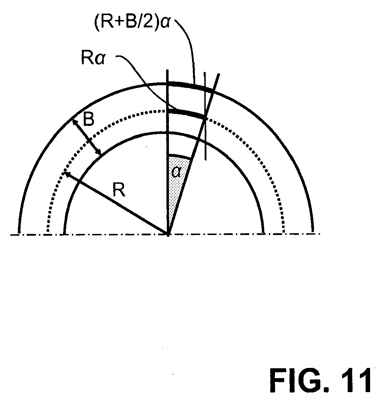

Anhand von Figur 11 wird nun noch eine elastische Dehnung dargestellt, die für den gecrimpten Zustand von Bedeutung ist. Im gecrimpten Zustand muss der Balken (Quersteg 32) maximal gekrümmt werden, um den Durchmesser des Stents zu minimieren. Die Größenordnungen der Dehnungen, die dabei auftreten, ergeben sich aus der in Figur 11 gezeigten Skizze. Beim Biegen eines Balkens (Breite B) mit dem Radius R wird jedes Stück der Außenkontur von seiner ursprünglichen Länge R·α auf die Länge (R + B/2) · a gedehnt. Die relative Dehnung ist also ε = ΔL / L = (B/2) / R. Im idealisierten Fall einer Biegung zum Halbkreis entspricht die Gesamtlänge L des Balkens gerade L = R·π. Das Material muss also eine Dehnung von

$$\varepsilon = (B/2) / R = ( \pi / 2 ) \cdot (B / L)$$

erlauben. Das Verhältnis B/L liegt bei typischen Steggeometrien deutlich unterhalb von 1/10, so dass diese Dehnung etwa in der Größenordnung von 10 % liegt. Übliche Werkstoffe (316L, CoCr, auch Mg) erreichen solche Werte nur durch plastische Verformung. Dies ist an sich noch kein prinzipieller Ausschlussgrund, jedoch ist dann besonders darauf zu achten, dass bei dieser Verformung (bzw. komplementär bei entsprechender Streckung zur Dilatation) keine Kräfte auftreten, die die Belastbarkeit des "schwächeren" Elements der Grundstruktur (z.B. Längssteg 36) übersteigen.

Wird als Werkstoff eine Gedächtnismetall-Legierung, beispielsweise der Werkstoff Nitinol, verwendet, kann diese Verformung allein durch elastische Dehnung des Werkstoffs der Grundstruktur erreicht werden, da die maximalen elastischen Dehnungen derartiger Werkstoffe die Größenordnung von 10% erreichen können. Der Stent wird damit selbstexpandierendes Verhalten zeigen, d.h. das Crimpen/Dilatieren stellt in dieser Hinsicht an das Element (z.B. Längssteg 36) keine besonderen Anforderungen. Die Verwendung einer Gedächtnismetall-Legierung als Grundmaterial erscheint daher im Zusammenhang mit der Erfindung vorteilhaft.

Anhand einer quantitativen Finite-Elemente-Analyse (FEM-Analyse) können zur Verwirklichung der angegebenen Grundstrukturen weitere Details geklärt werden, die sich auf das Design der jeweils zu realisierenden Endoprothese auswirken. Derartige praktisch relevante Details sind:

- Es kann anhand der FEM-Analyse berechnet werden, ob sich aus den transienten Konfigurationen eines Grundstruktur-Elements im Übergang von der Biege- zur Streckbeanspruchung engere Grenzen für die Stabilität ergeben.

- Ohne weitere stabilisierende Designelemente kann das Design dazu tendieren, sich bei Belastung unsymmetrisch zu verformen, so dass zwei benachbarte Elemente gemeinsam in eine nicht gewünschte Richtung, z.B. gemeinsam in die axiale (evtl. auch radiale) Richtung ausweichen. Es kann im Einzelnen berechnet werden, welche weiteren Stabilisierungen (z.B. Längsverbinder) nötig sind, um das auszuschließen. Ferner kann geklärt werden, ob benachbarte Segmentringe besser gleich- oder gegenphasig oder versetzt angeordnet werden sollten.

- Es kann aus den Berechnungen ferner ermittelt werden, ob der Elastizitätsbereich von Nitinol oder anderen Formgedächtnismetall-Legierungen für den erfindungsgemäßen Funktionsumfang ausreicht (ggf. mit Designanpassungen).

- Es kann im Einzelnen berechnet werden, ob für Elemente der Grundstruktur im gecrimpten Zustand eine S-Form oder eine gerade Form günstiger ist.

- Ferner kann ermittelt werden, ob für Elemente der Grundstruktur im Normalzustand eine Krümmung in eine bestimmte Richtung günstiger ist als eine gerade oder als eine andersherum gekrümmte Form.

- Es ist weiter zu berechnen, wie die Verbindungsstelle zwischen zwei Stegen gestaltet werden muss, damit die zu erwartenden Belastungen nicht zu lokaler Überlastung (Materialermüdung, Mikrorisse) führen.

- Ferner kann anhand der FEM-Berechnungen geklärt werden, ob eine Realisierung des erfindungsgemäßen Designs aus konventionellen Werkstoffen (316L, CoCr, Mg) grundsätzlich möglich ist. Hierbei muss die Frage geklärt werden, ob das Design auch so ausgeführt werden kann, dass die Elemente der Grundstruktur nicht nur bei Kompression, sondern auch bei Expansion eine hohe Zugkraft übertragen können (Dies ist eine notwendige Voraussetzung für den Einsatz von Werkstoffen, bei denen die Verformung des Elements vom minimalen ("gecrimpten") zum ("expandierten") Normalzustand prinzipiell nur plastisch erfolgen kann. Entsprechende Stents sind nicht selbstexpandierend, sondern müssen mit einem Ballon dilatiert werden.)

Bezugszeichenliste:

5    Längsrichtung
10    Steg
11    Innenseite
12    V-förmige Kerbe
13    Abschnitt der Seitenwand
14    Kerbe
15    Halsabschnitt
16    zylinderförmiger Abschnitt
18    Kerbe
19    quaderförmiger Abschnitt
21    Anschlagelement
25    Absatz/Vertiefung
26    Vertiefung
30    Längssteg
32    Quersteg
34    Begrenzungssteg
35    Verbindungsstelle
36    Längssteg
37    Verbindungsstelle
40    Quersteg
42    Anschlussstelle
44    Konnektorsteg

**Patentansprüche**

**1.** Endoprothese, vorzugsweise intraluminale Endoprothese, mit einer vorzugsweise hohlzylinderförmig ausgebildeten Grundstruktur, vorzugsweise als Grundgitter ausgestaltet, wobei im expandierten Zustand ein durch die Grundstruktur eingeschlossenes Innenvolumen durch die Elastizität der Grundstruktur veränderbar ist, wobei die Grundstruktur hinsichtlich ihres Kompressionsverhaltens einen Normalzustand und einen Zustand erheblicher Kompression einnehmen kann, wobei in dem Zustand erheblicher Kompression die Elastizität der Grundstruktur signifikant gegenüber der Elastizität im Normalzustand verringert ist, wobei sich der Zustand erheblicher Kompression durch Unterschreiten eines Innendurchmesser-Schwellwerts der Grundstruktur oder durch Überschreiten eines Kompressionsdruck-Schwellwerts auszeichnet, wobei der Innendurchmesser-Schwellwert vorzugsweise etwa 75% eines Nominal-Innendurchmessers beträgt und wobei der Kompressionsdruck-Schwellwert vorzugsweise etwa 0,2 bar, besonders bevorzugt etwa 0,1 bar und noch weiter bevorzugt etwa 0,075 bar beträgt, **dadurch gekennzeichnet, dass** der elastische Widerstand der Endoprothese im Zustand erheblicher Kompression mindestens das Zehnfache des elastischen Widerstands im Normalzustand beträgt.

**2.** Endoprothese nach Anspruch 1, **dadurch gekennzeichnet, dass** die im Zustand erheblicher Kompression in die Endoprothese eingeleiteten Kräfte überwiegend einen einachsigen Spannungszustand von zumindest einem Teil der Elemente der Endoprothese bewirken.

**3.** Endoprothese nach einem der vorhergehenden Ansprüche, **dadurch gekennzeichnet, dass** der Übergang von der Elastizität im Normalzustand zur Elastizität im Zustand erheblicher Kompression kontinuierlich verläuft.

**4.** Endoprothese nach einem der vorhergehenden Ansprüche, **dadurch gekennzeichnet, dass** die Grundstruktur mindestens überwiegend aus einer Formgedächtnismetall-Legierung, vorzugsweise Nitinol, besteht.

**5.** Endoprothese nach einem der vorhergehenden Ansprüche, **dadurch gekennzeichnet, dass** die Grundstruktur Mittel (13; 21, 10) aufweist, die bei Unterschreiten des bestimmten, vorgegebenen Innendurchmesser-Schwellwerts aneinander anliegen.

**6.** Endoprothese nach Anspruch 5, **dadurch gekennzeichnet, dass** die Mittel als eine Kerbe oder mehrere Kerben (12, 14, 18) in mindestens einem Steg (10) der Grundstruktur ausgebildet sind, wobei Wandabschnitte (13) der Kerbe oder der Kerben (12, 14, 18) bei Unterschreiten oder Überschreiten eines bestimmten, vorgegebenen Krümmungsradius des jeweiligen Stegs (10) im Zustand erheblicher Kompression derart aneinander anliegen, dass sie einer weiteren Verringerung des mittleren Krümmungsradius des jeweiligen Stegs (10) bzw. einer weiteren Vergrößerung des mittleren Krümmungsradius des jeweiligen Stegs entgegenwirken.

7. Endoprothese nach Anspruch 5, **dadurch gekennzeichnet, dass** die Mittel als ein von einem jeweiligen Steg (10) abstehendes, im Wesentlichen quer zur Längsrichtung der Endoprothese verlaufendes Anschlagelement (21) ausgebildet sind, wobei jedes Anschlagelement (21) jeweils bei Unterschreiten des bestimmten, vorgegebenen Innendurchmesser-Schwellwerts an einen jeweils gegenüber liegenden Steg (10) anschlägt.

8. Endoprothese nach einem der Ansprüche 5 bis 7, **dadurch gekennzeichnet, dass** die Mittel Formschlussmittel (21, 25, 26) aufweisen, die im Zustand erheblicher Kompression einen Formschluss ausbilden.

9. Endoprothese nach einem der vorhergehenden Ansprüche, **dadurch gekennzeichnet, dass** die Grundstruktur im Wesentlichen quer zur Längsrichtung der Endoprothese verlaufende, im Normalzustand vorzugsweise gekrümmte Querstege (32) sowie im Wesentlichen in Längsrichtung verlaufende Längsstege (30) aufweist, wobei die Querstege (32) und die Längsstege (30) miteinander verbunden und quer zur Längsrichtung der Endoprothese abwechselnd hintereinander angeordnet sind, wobei parallel zu einem Quersteg (32) zwischen je zwei Verbindungsstellen jeweils ein Begrenzungssteg (34) angeordnet ist, welcher im Zustand erheblicher Kompression, wenn der Krümmungsradius des Querstegs (32) oberhalb eines bestimmten, vordefinierten Krümmungsradius-Schwellwerts liegt, einer weiteren Vergrößerung des Krümmungsradius des Querstegs (32) entgegenwirkt.

10. Endoprothese nach einem der vorhergehenden Ansprüche, **dadurch gekennzeichnet, dass** die Grundstruktur Maschen aufweist, die durch jeweils zwei im Wesentlichen quer zur Längsrichtung der Endoprothese verlaufende Querstege (40) ausgebildet sind, welche an ihren Enden miteinander verbunden sind, wobei beide Querstege (40) zusätzlich mit mindestens einem im Wesentlichen in Längsrichtung der Endoprothese verlaufenden Konnektorsteg (44) verbunden sind, der im Zustand erheblicher Kompression einer weiteren Vergrößerung des Abstands zwischen beiden Querstegen (40) entgegenwirkt.

11. Endoprothese nach einem der vorhergehenden Ansprüche, **dadurch gekennzeichnet, dass** die Grundstruktur durch abwechselnd in die Richtung quer zur Längsrichtung der Endoprothese hintereinander angeordnete und miteinander verbundene Querstege (32) und Begrenzungsstege (36) ausgebildet ist, wobei die Querstege (32) im Normalzustand im Wesentlichen quer zur Längsrichtung und die Begrenzungsstege (36) im Normalzustand zumindest abschnittsweise im Wesentlichen in Längsrichtung der Endoprothese verlaufen, wobei die Begrenzungsstege (36) mit zunehmender Kompression derart drehbar sind, dass sie zunehmend quer zur Längsrichtung verlaufen, wobei die Elastizität der Begrenzungsstege (36) bei zunehmender Verdrehung abnimmt, wobei im Normalzustand die Federkonstante a der Querstege (32) größer ist als die Federkonstante b der Begrenzungsstege (36), vorzugsweise a > 10 · b, und im Zustand erheblicher Kompression die Zugfestigkeit der Begrenzungsstege größer ist als die zur Verbiegung der Querstege nötige Kraft.

12. Endoprothese nach einem der Ansprüche 8 bis 10, **dadurch gekennzeichnet, dass** die Begrenzungsstege (34, 36) oder die Konnektorstege (44) eine kleinere Breite als die Querstege (32, 40) aufweisen, wobei die Breite der Begrenzungsstege (34, 36) oder der Konnektorstege (44) vorzugsweise kleiner als 1/3 der Breite der Querstege (32, 40) ist.

13. Endoprothese nach einem der vorhergehenden Ansprüche, **dadurch gekennzeichnet, dass** die Grundstruktur in einem gecrimpten Zustand einen Innendurchmesser aufweist, der signifikant kleiner ist als der Innendurchmesser der Endoprothese im Zustand erheblicher Kompression.

**Claims**

1. An endoprosthesis, preferably an intraluminal endoprosthesis, having a basic structure which is preferably formed in the shape of a hollow cylinder and which is preferably configured as a basic lattice, wherein, in an expanded state, an inner volume enclosed by the basic structure can be changed by means of the elasticity of the basic structure, wherein the basic structure can assume a normal state and a state of significant compression with regard to its compression behaviour, wherein, in the state of significant compression, the elasticity of the basic structure is significantly reduced as compared with the elasticity in the normal state, wherein the state of significant compression is **characterised by** the fact that an inside diameter threshold value of the basic structure is not reached, or that a compression pressure threshold value is exceeded, wherein the inside diameter threshold value preferably amounts to about 75% of a nominal inside diameter, and wherein the compression pressure threshold value amounts to preferably about 0.2 bar, particularly preferably about 0.1 bar, and even more preferably about 0.075 bar, **characterised in that** the elastic resistance of the endoprosthesis in the state of significant compression amounts to at least ten times the elastic resistance in the normal state.

2. The endoprosthesis according to Claim 1, **characterised in that** the forces introduced into the endoprosthesis in the state of significant compression predominantly bring about a single-axis stress state of at least some of the elements of the endoprosthesis.

3. The endoprosthesis according to one of the preceding claims, **characterised in that** the transition from the elasticity in the normal state to the elasticity in the state of significant compression proceeds continuously.

4. The endoprosthesis according to one of the preceding claims, **characterised in that** the basic structure consists at least predominantly of a shape-memory metal alloy, preferably nitinol.

5. The endoprosthesis according to one of the preceding claims, **characterised in that** the basic structure has means (13; 21, 10) that lie against one another if the specific, predetermined inside diameter threshold value is not reached.

6. The endoprosthesis according to Claim 5, **characterised in that** the means are configured as a notch or multiple notches (12, 14, 18) in at least one crosspiece (10) of the basic structure, wherein wall portions (13) of the notch or notches (12, 14, 18) lie against one another if a specific, predetermined radius of curvature of the respective crosspiece (10) is not reached or is exceeded in the state of significant compression, in such a manner that they counteract a further reduction in the average radius of curvature of the respective crosspiece (10) or, respectively, a greater increase in the average radius of curvature of the respective crosspiece.

7. The endoprosthesis according to Claim 5, **characterised in that** the means are configured as a contact element (21) that projects from a respective crosspiece (10) and runs substantially transverse to the longitudinal direction of the endoprosthesis, wherein each contact element (21) impacts against a respective opposite crosspiece (10) if the specific, predetermined inside diameter threshold value is not reached.

8. The endoprosthesis according to one of Claims 5 to 7, **characterised in that** the means have form-fit means (21, 25, 26), which form a form fit in the state of significant compression.

9. The endoprosthesis according to one of the preceding claims, **characterised in that** the basic structure has transverse crosspieces (32) that run substantially transverse to the longitudinal direction of the endoprosthesis and are preferably curved in the normal state, as well as longitudinal crosspieces (30) that run substantially in the longitudinal direction, wherein the transverse crosspieces (32) and the longitudinal crosspieces (30) are connected with one another and disposed alternately one behind the other transverse to the longitudinal direction of the endoprosthesis, wherein a restriction crosspiece (34), in each instance, is disposed parallel to a transverse crosspiece (32) arranged in each instance between two connection locations, which restriction crosspiece, in the state of significant compression, counteracts a further increase in the radius of curvature of the transverse crosspiece (32) if the radius of curvature of the transverse (32) crosspiece lies above a specific, predefined radius of curvature threshold value.

10. The endoprosthesis according to one of the preceding claims, **characterised in that** the basic structure has meshes that are each formed by two transverse crosspieces (40) that run substantially transverse to the longitudinal direction of the endoprosthesis and are connected with one another at their ends, wherein the two transverse crosspieces (40) are additionally connected with at least one connector crosspiece (44), which runs substantially in the longitudinal direction of the endoprosthesis and, in the state of significant compression, counteracts a further increase in the distance between the two transverse crosspieces (40).

11. The endoprosthesis according to one of the preceding claims, **characterised in that** the basic structure is formed by transverse crosspieces (32) and restriction crosspieces (36) that are alternately disposed one behind the other in the direction transverse to the longitudinal direction of the endoprosthesis and are connected with one another, wherein the transverse crosspieces (32), in the normal state, run substantially transverse to the longitudinal direction, and the restriction crosspieces (36), in the normal state, run substantially in the longitudinal direction of the endoprosthesis at least in portions, wherein the restriction crosspieces (36) can be rotated, with increasing compression, in such a manner that they increasingly run transverse to the longitudinal direction, wherein the elasticity of the restriction crosspieces (36) decreases with increasing rotation, wherein, in the normal state, the spring constant a of the transverse crosspieces (32) is greater than the spring constant b of the restriction crosspieces (36), preferably a>10·b, and, in the state of significant compression, the tensile strength of the restriction crosspieces is greater than the force required to bend the transverse crosspieces.

12. The endoprosthesis according to one of Claims 8 to 10, **characterised in that** the restriction crosspieces (34, 36) or the connector crosspieces (44) have a smaller width than the transverse crosspieces (32, 40), wherein the width of the restriction crosspieces (34, 36) or the connector crosspieces (44) is preferably less than 1/3 of the width of the transverse crosspieces (32, 40).

13. The endoprosthesis according to one of the preceding claims, **characterised in that** the basic structure, in a crimped state, has an inside diameter that is significantly smaller than the inside diameter of the endoprosthesis in the state of significant compression.


**Revendications**

1. Endoprothèse, de préférence, endoprothèse intraluminale, avec de préférence une structure de base conçue en forme de cylindre creux, de préférence conçue en tant que grille de base, où, dans l'état en expansion, un volume intérieur enclavé par la structure de base peut être modifié par l'élasticité de la structure de base, où la structure de base, en ce qui concerne son comportement sous compression, peut adopter un état normal et un état de compression substantielle, où, dans l'état de compression substantielle, l'élasticité de la structure de base est diminuée de manière significative par rapport à l'élasticité à l'état normal, où l'état de compression substantielle se **caractérise par** une valeur inférieure à une valeur de seuil du diamètre intérieur de la structure de base ou par une valeur supérieure à une valeur de seuil de la pression de compression, où la valeur de seuil du diamètre intérieur se situe à environ 75% d'un diamètre intérieur nominal et où la valeur de seuil de la pression de compression se situe de préférence à environ 0,2 bar, notamment de préférence à environ 0,1 bar, et de manière encore préférée de préférence à environ 0,075 bar, **caractérisée en ce que** la résistance élastique de l'endoprothèse à l'état de compression substantielle se situe à un niveau égal à au moins dix fois la résistance élastique dans l'état normal.

2. Endoprothèse selon la revendication 1, **caractérisée en ce que** les forces emmagasinées dans l'endoprothèse à l'état de compression substantielle provoquent principalement un état de tension sur un axe d'au moins une partie des éléments de l'endoprothèse.

3. Endoprothèse selon l'une des revendications précédentes, **caractérisée en ce que** la transition de l'élasticité à l'état normal vers l'élasticité à l'état de compression substantielle se déroule de manière continue.

4. Endoprothèse selon l'une des revendications précédentes, **caractérisée en ce que** la structure de base est constituée au moins principalement d'un alliage à mémoire de forme, de préférence de Nitinol.

5. Endoprothèse selon l'une des revendications précédentes, **caractérisée en ce que** la structure de base présente des moyens (13 ; 21, 10) qui, lors d'une valeur inférieure à la valeur de seuil du diamètre intérieur définie prédéterminée viennent en butée les uns dans les autres.

6. Endoprothèse selon la revendication 5, **caractérisée en ce que** les moyens sont conçus sous la forme d'une entaille ou de plusieurs entailles (12, 14, 18) dans au moins un barreau (10) de la structure de base, où, lors d'une valeur inférieure à un seuil ou d'une valeur supérieure à un seuil d'un rayon de courbure défini prédéterminé du barreau (10) en question, les parties des parois (13) de l'entaille ou des entailles (12, 14, 18) sont en butée les unes dans les autres à l'état de compression substantielle de sorte qu'elles agissent à l'encontre d'une diminution ultérieure du rayon de courbure moyen du barreau (10) en question, respectivement, d'un agrandissement ultérieur du rayon de courbure moyen du barreau en question.

7. Endoprothèse selon la revendication 5, **caractérisée en ce que** les moyens sont constitués sous la forme d'un élément de butée (21) en saillie par rapport à un barreau (10) donné, s'étendant dans l'ensemble perpendiculairement par rapport à la direction longitudinale de l'endoprothèse, où chaque élément de butée (21) se met chaque fois en butée vis-à-vis de chaque barreau (10) situé en face lors d'une valeur inférieure à une valeur de seuil du diamètre intérieur donnée prédéterminée.

8. Endoprothèse selon l'une des revendications 5 à 7, **caractérisée en ce que** les moyens présentent des moyens de complémentarité de formes (21, 25, 26) qui forment un système par complémentarité des formes à l'état de compression substantielle.

9. Endoprothèse selon l'une des revendications précédentes, **caractérisée en ce que** la structure de base présente des barreaux transverses (32) s'étendant dans l'ensemble perpendiculaires à la direction longitudinale de l'endoprothèse, de préférence recourbées à l'état normal, ainsi que des barreaux longitudinaux (30) s'étendant dans l'ensemble en direction longitudinale, où les barreaux transverses (32) et les barreaux longitudinaux (30) sont reliés ensemble et sont disposés, successivement alternés, perpendiculairement par rapport à la direction longitudinale de l'endoprothèse, où un barreau de délimitation (34) est chaque fois disposé parallèle à un barreau transverse (32) entre chaque fois deux points de liaison, lequel, à l'état de compression substantielle, lorsque le rayon de courbure de la barre transversale (32) se situe à une valeur supérieure à une valeur de seuil de rayon de courbure définie, prédéterminée, agit à l'encontre d'un agrandissement ultérieur du rayon de courbure du barreau transverse (32).

10. Endoprothèse selon l'une des revendications précédentes, **caractérisée en ce que** la structure de base présente des mailles qui sont formées par chaque fois deux barreaux transverses (40) s'étendant dans l'ensemble perpendiculairement par rapport à la direction longitudinale de l'endoprothèse, lesquels sont reliés ensemble à leurs extrémités, où les deux barreaux transverses (40) sont reliés de manière complémentaire avec au moins un barreau de connexion (44) s'étendant dans l'ensemble dans la direction longitudinale de l'endoprothèse, qui, à l'état de compression substantielle, agit contre un agrandissement ultérieur de la distance entre les deux barreaux transverses (40).

11. Endoprothèse selon l'une des revendications précédentes, **caractérisée en ce que** la structure de base est formée de barreaux transverses (32) et de barreaux de délimitation (36) disposés les uns derrière les autres en alternance dans la direction perpendiculaire par rapport à la direction longitudinale de l'endoprothèse et reliés ensemble, où les barreaux transverses (32), à l'état normal, s'étendent dans l'ensemble perpendiculairement par rapport à la direction longitudinale et les barreaux de délimitation (36) à l'état normal s'étendent au moins partiellement dans l'ensemble dans la direction longitudinale de l'endoprothèse, où les barres de délimitation (36) peuvent être mis en rotation avec une compression en augmentation de telle manière qu'ils s'étendent de manière croissante perpendiculairement par rapport à la direction longitudinale, où l'élasticité des barreaux de délimitation (36) diminue pour une rotation croissante, où, à l'état normal, la constante élastique a des barreaux transverses (32) est supérieure à la constante élastique b des barreaux de délimitation (36), de préférence a > 10 b, et, à l'état de compression substantielle, la résistance en traction des barreaux de délimitation est supérieure à la force nécessaire pour la flexion des barreaux transverses.

12. Endoprothèse selon l'une des revendications 8 à 10, **caractérisée en ce que** les barreaux de délimitation (34, 36) ou les barreaux de connexion (44) présentent une largeur inférieure à celle des barreaux transverses (32, 40), où la largeur des barreaux de délimitation (34, 36) ou des barreaux de connexion (44) est de préférence inférieure à 1/3 de la largeur des barreaux transverses (32, 40).

13. Endoprothèse selon l'une des revendications précédentes, **caractérisée en ce que** la structure de base dans un état serti présente un diamètre intérieur qui est significativement inférieur au diamètre intérieure de l'endoprothèse à l'état de compression substantielle.

**FIG. 1A**

**FIG. 1B**

**FIG. 2**

**FIG. 3**

FIG. 6A

FIG. 6B

FIG. 6C

FIG. 6D

EP 2 332 499 B1

FIG. 4A

FIG. 4B

FIG. 4C

FIG. 4D

FIG. 5A

FIG. 5B

EP 2 332 499 B1

FIG. 7C

FIG. 7B

FIG. 7A

FIG. 8A

FIG. 8B

FIG. 8C

FIG. 8D

FIG. 9

$F_B$

B

L

FIG. 10

B

L

$F_Z$

FIG. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1642551 A1